# EUROPEAN PATENT APPLICATION

(11) **EP 1 178 117 A1**
(43) Date of publication of application: **06.02.2002**
(21) Application number: 00202750.6
(22) Date of filing: 02.08.2000
(51) Int. Cl.: C12N 15/87, C12N 15/88, C12N 15/86, A61K 48/00

(54) **Targeting through integrins**

(71) Applicant: Erasmus Universiteit Rotterdam, 3015 GE Rotterdam (NL)
(72) Inventor: Scholte, Bob Johan, 3069 AT Rotterdam (NL)
(74) Representative: Prins, Adrianus Willem

(57) **Abstract**

The present invention relates to a gene delivery vehicle comprising a general nucleic acid binding domain and an integrin binding domain. Typically, the at least two different domains of the gene delivery vehicle are somehow linked. Said gene delivery vehicle may be used to deliver a therapeutic molecule to a cell exposing integrin, thereby at least in part enhancing the efficacy of gene delivery using integrin receptors. In one embodiment, an integrin binding domain of the invention is derived from the C-terminal region of the *Yersinia pseudotuberculosis* invasin or a functional homologue thereof.

### Annex to the application documents - subsequently filed sequences listing

SEQUENCE LISTING

<110> Erasmus Universiteit Rotterdam

<120> Targeting through integrins

<130> P52462EP00

<140> EP 00202750.6
<141> 2000-08-02

<160> 6

<170> PatentIn Ver. 2.1

<210> 1
<211> 57
<212> DNA
<213> Artificial Sequence

<220>
<223> Description of Artificial Sequence: olionucleotide

<400> 1
catggccagc ccaaaacgca gcccaaaacg cagcccaaaa cgcagcccaa aacgcgg    57

<210> 2
<211> 57
<212> DNA
<213> Artificial Sequence

<220>
<223> Description of Artificial Sequence:
      oligonucleotide

<400> 2
gatcccgcgt tttgggctgc gttttgggct gcgttttggg ctgcgttttg ggctggc    57

<210> 3
<211> 17
<212> DNA
<213> Artificial Sequence

<220>
<223> Description of Artificial Sequence: PCR primer

<400> 3
attcccggta cctacgc

<210> 4
<211> 19
<212> DNA
<213> Artificial Sequence

<220>
<223> Description of Artificial Sequence: PCR primer

<400> 4
gaatgctata ttgacagcg                                    19


<210> 5
<211> 855
<212> DNA
<213> Artificial Sequence

<220>
<223> Description of Artificial Sequence: (SPRK)4 inv

<220>
<221> CDS
<222> (1)..(852)

<400> 5
atg cac cat cat cat cat cat tct tct ggt ctg gtg cca cgc ggt tct   48
Met His His His His His His Ser Ser Gly Leu Val Pro Arg Gly Ser
 1               5                  10                  15

ggt atg aaa gaa acc gct gct gct aaa ttc gaa cgc cag cac atg gac   96
Gly Met Lys Glu Thr Ala Ala Ala Lys Phe Glu Arg Gln His Met Asp
            20                  25                  30

agc cca gat ctg ggt acc gac gac gac gac aag gcc atg gcc agc cca   144
Ser Pro Asp Leu Gly Thr Asp Asp Asp Asp Lys Ala Met Ala Ser Pro
        35                  40                  45

aaa cgc agc cca aaa cgc agc cca aaa cgc agc cca aaa cgc ggg atc   192
Lys Arg Ser Pro Lys Arg Ser Pro Lys Arg Ser Pro Lys Arg Gly Ile
    50                  55                  60

cca acc tat ctg agc gaa gat gaa ctg aaa gcc gcc gaa gcc gcc ttc   240
Pro Thr Tyr Leu Ser Glu Asp Glu Leu Lys Ala Ala Glu Ala Ala Phe
65                  70                  75                  80

aaa cgc cac aac cca acc gaa ttc ggc tta ttc ccg gta cct acg ctg   288
Lys Arg His Asn Pro Thr Glu Phe Gly Leu Phe Pro Val Pro Thr Leu
                85                  90                  95

acc ggt att ctg gtt aac ggg caa aat ttc gct acg gat aaa ggg ttc   336
Thr Gly Ile Leu Val Asn Gly Gln Asn Phe Ala Thr Asp Lys Gly Phe
            100                 105                 110

ccg aaa acg atc ttt aaa aac gcc aca ttc cag tta cag atg gat aac   384
Pro Lys Thr Ile Phe Lys Asn Ala Thr Phe Gln Leu Gln Met Asp Asn
        115                 120                 125

gat gtt gct aat aat act cag tat gag tgg tcg tcg tca ttc aca ccc   432
Asp Val Ala Asn Asn Thr Gln Tyr Glu Trp Ser Ser Ser Phe Thr Pro
    130                 135                 140

aat gta tcg gtt aac gat cag ggt cag gtg acg att acc tac caa acc   480
Asn Val Ser Val Asn Asp Gln Gly Gln Val Thr Ile Thr Tyr Gln Thr
145                 150                 155                 160

tat agc gaa gtg gct gtg acg gcg aaa agt aaa aaa ttc cca agt tat   528
Tyr Ser Glu Val Ala Val Thr Ala Lys Ser Lys Lys Phe Pro Ser Tyr
                165                 170                 175

tcg gtg agt tat cgg ttc tac cca aat cgg tgg ata tac gat ggc ggc   576
Ser Val Ser Tyr Arg Phe Tyr Pro Asn Arg Trp Ile Tyr Asp Gly Gly
            180                 185                 190

act tcg ctg gta tcg agt ctc gag gcc agc aga caa tgc caa ggt tca   624
Thr Ser Leu Val Ser Ser Leu Glu Ala Ser Arg Gln Cys Gln Gly Ser
        195                 200                 205

gat atg tct gcg gtt ctt gaa tcc tca cgt gca acc aac gga acg cgt   672
Asp Met Ser Ala Val Leu Glu Ser Ser Arg Ala Thr Asn Gly Thr Arg
    210                 215                 220

gcg cct gac ggg aca ttg tgg ggc gag tgg ggg agc ttg acc gcg tat   720
Ala Pro Asp Gly Thr Leu Trp Gly Glu Trp Gly Ser Leu Thr Ala Tyr
225                 230                 235                 240

agt tct gat tgg caa tct ggc gaa tat tgg gtc aaa aag acc agt acg   768
Ser Ser Asp Trp Gln Ser Gly Glu Tyr Trp Val Lys Lys Thr Ser Thr
                245                 250                 255

gat ttt gaa acc atg aat atg gac acc ggc gcg ctg gta caa ggg cct   816
Asp Phe Glu Thr Met Asn Met Asp Thr Gly Ala Leu Val Gln Gly Pro
            260                 265                 270

gca tac ttg cgg ttc ccg ctc tgt gcg ctg tca ata tag               855
Ala Tyr Leu Arg Phe Pro Leu Cys Ala Leu Ser Ile
        275                 280

<210> 6
<211> 284
<212> PRT
<213> Artificial Sequence
<223> Description of Artificial Sequence: (SPRK)4 inv

<400> 6
Met His His His His His His Ser Ser Gly Leu Val Pro Arg Gly Ser
1               5                   10                  15

Gly Met Lys Glu Thr Ala Ala Ala Lys Phe Glu Arg Gln His Met Asp
            20                  25                  30

Ser Pro Asp Leu Gly Thr Asp Asp Asp Asp Lys Ala Met Ala Ser Pro
        35                  40                  45

Lys Arg Ser Pro Lys Arg Ser Pro Lys Arg Ser Pro Lys Arg Gly Ile
    50                  55                  60

Pro Thr Tyr Leu Ser Glu Asp Glu Leu Lys Ala Ala Glu Ala Ala Phe
65                  70                  75                  80

Lys Arg His Asn Pro Thr Glu Phe Gly Leu Phe Pro Val Pro Thr Leu
                85                  90                  95

Thr Gly Ile Leu Val Asn Gly Gln Asn Phe Ala Thr Asp Lys Gly Phe
            100                 105                 110

Pro Lys Thr Ile Phe Lys Asn Ala Thr Phe Gln Leu Gln Met Asp Asn
        115                 120                 125

Asp Val Ala Asn Asn Thr Gln Tyr Glu Trp Ser Ser Ser Phe Thr Pro
    130                 135                 140

Asn Val Ser Val Asn Asp Gln Gly Gln Val Thr Ile Thr Tyr Gln Thr
145                 150                 155                 160

Tyr Ser Glu Val Ala Val Thr Ala Lys Ser Lys Lys Phe Pro Ser Tyr
                165                 170                 175

Ser Val Ser Tyr Arg Phe Tyr Pro Asn Arg Trp Ile Tyr Asp Gly Gly
            180                 185                 190

Thr Ser Leu Val Ser Ser Leu Glu Ala Ser Arg Gln Cys Gln Gly Ser
        195                 200                 205

Asp Met Ser Ala Val Leu Glu Ser Ser Arg Ala Thr Asn Gly Thr Arg
    210                 215                 220

Ala Pro Asp Gly Thr Leu Trp Gly Glu Trp Gly Ser Leu Thr Ala Tyr
225                 230                 235                 240

Ser Ser Asp Trp Gln Ser Gly Glu Tyr Trp Val Lys Lys Thr Ser Thr
                245                 250                 255

Asp Phe Glu Thr Met Asn Met Asp Thr Gly Ala Leu Val Gln Gly Pro
            260                 265                 270

Ala Tyr Leu Arg Phe Pro Leu Cys Ala Leu Ser Ile
        275                 280

## Description

The present invention relates to targeted therapeutic molecules and/or complexes. In particular it relates to delivery of therapeutic molecules to certain cells. In particular the invention relates to delivering therapeutic molecules to cells exposing integrins. Preferably the molecule to be delivered is a nucleic acid. The compounds capable of delivering said therapeutic nucleic acids are referred to as gene delivery vehicles and/or targeted (viral or non-viral) vectors.

### Introduction

Targeted non-viral vectors have great potential in gene therapy.¹⁻³ Delivery of the therapeutic gene through receptor mediated endocytosis (RME), specifically into the appropriate cell type is a major advantage of such vectors. This approach generally involves coupling a ligand of a cellular receptor or another molecule associated with the surface of the cell to be targeted, to a DNA binding element, usually a positively charged polymer such as poly-L-lysine,⁴ or Polyethylenimine.⁵⁻⁷ The polymer serves several important purposes. It condenses the DNA to a size suitable for RME, and it plays a role in the escape from the endosomal compartment. Several examples of ligand-polymer conjugates have been described in literature, featuring transferrin,^{8,9} asialo-glycoprotein receptor ligands,¹⁰ immunoglobulins, or serpin.² Upon mixing the ligand-polymer conjugate with plasmid DNA, the two elements form a complex, which enters the cells, typically by RME.
According to the invention the gene delivery vehicles are targeted to integrins. Integrins are dimers of two complementary transmembrane proteins, a- and b- chain, which form a binding-site for extracellular ligands. The ligand specificity is determined by the particular combination of α and β chains. Integrins are ubiquitously expressed by many cell types and are usually associated with matrix proteins in the basal region of polarized cells. However, these interactions are dynamic, in particular during migration and repair of damaged tissue. The intracellular integrin domains interact with signaling pathways that affect cellular motility and growth. Its affinity for an extra-cellular substrate depends on its state of conformational activation. Tumours have been successfully imaged with ⁹⁹Tc-labeled integrin-binding peptides,¹⁴ indicating a significantly increased availability of integrins in this tissue. Apical expression of β1 integrins has been observed on epithelial cells during inflammation, wound healing and ischaemia.¹⁵⁻¹⁷ Endothelial cells also express apical β1 integrins, which are involved in interactions with leukocytes when activated during the process of inflammation.¹⁸ In conclusion, β1 integrins are available to external ligands, especially during clinically relevant conditions. This makes them an interesting target for vector delivery. According to the invention integrin binding domains are applied as a part of the gene delivery vehicle conferring specificity. According to one embodiment such a binding domain is derived from invasin or functional homologues thereof.
Several pathogens, including viruses and bacteria use integrin receptors to invade their target cells. One of these is *Yersinia pseudotuberculosis,* which makes the outer-membrane protein invasin. This protein binds with high affinity to β1 type integrins and thus mediates phagocytosis of the bacteria. The integrin-binding property resides in the carboxy-terminal part of the protein, in particular the terminal 192 amino-acid loop structure.¹⁹ This protein domain can be used to induce uptake of material by integrin expressing cells,²⁰ as an alternative to synthetic peptides. Peptides that contain the integrin-binding RGD amino acid motif substantially improve transfection efficiency *in vitro,* when coupled chemically to a cationic polymer.^{21,22} However, proof that such preparations are able to deliver DNA efficiently to integrin exposing cells *in vivo* has not been presented yet. Major problems in the formulation of a compound that can be used systemically are caused by non-specific interactions with blood-components, and the tendency of the complexes to dissociate or aggregate. Moreover, chemical coupling of a ligand to a polymer is generally inefficient, difficult to reproduce, and results in a random conjugation product. An alternative provided by the present invention is to combine the DNA binding- and ligand elements in e.g. a single modular recombinant protein.
Thus the invention provides a gene delivery vehicle comprising a general nucleic acid binding domain and an integrin binding domain. A general nucleic acid binding domain is defined as a preferably proteinaceous domain that can bind to many different nucleic acid sequences, i.e. which is not limited in binding to a single or only a few sequences. A nucleic acid may be DNA, RNA, PNA, single stranded, double stranded homoduplex or heteroduplex, multimer, circular or linear. An integrin binding domain is defined as a preferably proteinaceous domain that can specifically recognise an integrin, typically in the context of the integrin being associated with the surface of a cell. Typically the at least two different domains of the gene delivery vehicle are somehow linked, so that the one side can bind the nucleic acid that is supposed to be delivered to the cell, whereas the other side can bind to the cell and preferably be internalised by the cell to deliver the nucleic acid to the cell. Incidentally, the integrin binding domains according to the invention may also be used to deliver other molecules then nucleic acids to cells. An easy way of physically linking an integrin binding domain and a nucleic acid binding domain to each other, when both are proteinaceous, is through the production of a fusion-protein. Such a fusion-protein is preferably a multidomain protein. Thus in a preferred embodiment the invention provides a gene delivery vehicle, which comprises a fusion-protein of said nucleic acid binding domain and said integrin binding domain. In one embodiment, said fusion-protein can be provided with a functionality of fusing membranes.
Preferably, the integrin binding domain binds β1-integrin. β1-integrins are made by many cell types, and are involved in cell matrix interactions (fibronectin receptor). In intact tissue, these integrins are normally not exposed free on the plasma-membrane. However, they become exposed and activated in damaged or remodelling tissues. Therefore, in situations where we observe a combination of cell migration (receptor exposure) and mitotic activity (nuclear transport of DNA), we can expect both efficient delivery and vector expression with a β1-integrin targeted delivery system. Clinically relevant examples of such a situation are:
- Damage to lung epithelium can occur during acute and chronic inflammation as in Cystic Fibrosis lung disease and Asthma. This is associated with β1 integrin exposure on epithelial cells.^{15,16,17} Delivery of therapeutic genes (CFTR, IL10) to damaged areas will be beneficial to the patient.
- Damage to the arterial wall after angioplasty is often associated with restenosis. Damaged vascular endothelium exposes β1 integrins,¹⁸ which would allow for targeted delivery of therapeutic gene (DNA, RNA) or bio-active drugs.
- Coronary or peripheral ischaemia after arterial blockage, is followed by a phase of cellular migration and repair.
   This is also associated with exposure of β1 integrins on both endothelial cells and underlying cardiomyocytes.
- Sites of tumor growth and tumor cell attachment are also possible targets of β1 integrin targeted complexes.¹⁴
- During wound healing and nerve regeneration, migrating and proliferating cells express β1 integrins.^{41,42}

For binding to integrin the preferred binding domain is derived from invasin or a functional homologue thereof. Thus in a further embodiment the invention provides a gene delivery vehicle, wherein said integrin binding domain is derived from the C-terminal region of the *Yersinia pseudotuberculosis* invasin or a functional homologue thereof. A functional homologue is defined as a molecule from another or the same species that has a similar binding domain for integrins as the invasin binding domain. Derived from such a binding domain means that the functionality is maintained (in kind not necessarily in amount), but that a number of e.g. amino acid residues are replaced by different amino acid residues.
For reasons of ease of manipulation, stability and such, as well as ease of presenting genetic information to cells, it is preferred that the nucleic acid to be delivered is DNA, especially double stranded DNA. Thus the invention provides in another embodiment a gene delivery vehicle wherein said nucleic acid binding domain is a DNA-binding domain, preferably a DNA-binding domain which is derived from a histon DNA-binding domain, in particular from (SPKR)₄ or a functional homologue thereof. Definitions of homologues and/or derivatives are the same as used before. When both domains are physically linked it is advantageous to avoid possible hindrance of the one binding domain for the other. Therefore separation through a linker is preferred. Thus the invention provides a gene delivery vehicle wherein said domains are connected through a rigid alpha-helical linker, preferably a gene delivery vehicle wherein said domains are coupled to said linker through flexible hinge sequences. The gene delivery vehicles according to the invention are intended for delivery of nucleic acids (herein generally referred to as genes) to cells. To that effect the delivery vehicle must be contacted with the nucleic acid of interest. This results in a complex of a gene delivery vehicle according to the invention, associated with a nucleic acid comprising a gene of interest to be delivered, which is yet another embodiment of the present invention. Associated is defined herein that there is some kind of binding between the relevant binding domain and its counterpart, typically the same kind of binding that occurs in nature. Genes (nucleic acids) of interest can comprise coding sequences coding for therapeutic genes such as factor 8, CFTR, etc or encoding cytokines or cytotoxic substances, but can also be cosuppressing agents or blocking agents such as antisense molecules and/or defective genes, etc. The gene of interest can be presented in any suitable format, but a plasmid format or a vector format is preferred.
Of course the complex must be presented to cells (in vitro or in vivo) in a suitable composition. Thus the invention also provides a composition for delivery of a gene of interest to a cell, comprising a complex according the invention and a suitable excipient. Typically the effectiveness in transfection of such a complex can be enhanced by the presence of a lysogenic lipid in the composition. Thus the invention further provides a composition further comprising a lysogenic lipid, preferably a lysogenic lipid composition which comprises DOTAP. Nucleic acids are somewhat fragile molecules, which can be protected by condensing them, e.g. on a polykationic polymer. It is thus preferred that such a kationic poymer or polykation is present in the compositions according to the invention. Thus the invention provides a composition further comprising a polykation on which the nucleic can condense. Preferably said polykation comprises polyethylene-imine or functional derivative and/or analogue thereof. In said composition said polykation may be present in associated form. However, said polykation may also be covalently linked to said general nucleic acid binding domain and/or said integrin binding domain.
Nucleic acids encoding the gene delivery vehicles of the invention are also part of the present invention. The gene delivery vehicle may be non-viral, but it can also be, or comprise, a viral gene delivery vehicle, in which case the nucleic acid encoding the gene delivery vehicle is typically a vector derived from a virus, such as an adenovirus, so that the gene delivery vehicle will be a viral particle, comprising an integrin binding domain, to which the nucleic acid to be delivered can be bound or otherwise incorporated.

### Detailed description.

Herein we describe the production in a bacterial expression system, and functional characterization of a novel modular protein, (SPKR)₄inv, designed to mediate integrin targeted gene delivery. (SPKR)₄inv spontaneously associates with plasmid DNA and binds to cellular integrins with high affinity. In combination with a cationic liposome (DOTAP) or a cationic polymer (PEI), efficient expression of marker gene in different cell lines was achieved. This modular fusion-protein system can be used as (part of) a gene delivery system targeted to cells exposing β1-integrin receptors.

### Results

### Construction of expression plasmids.

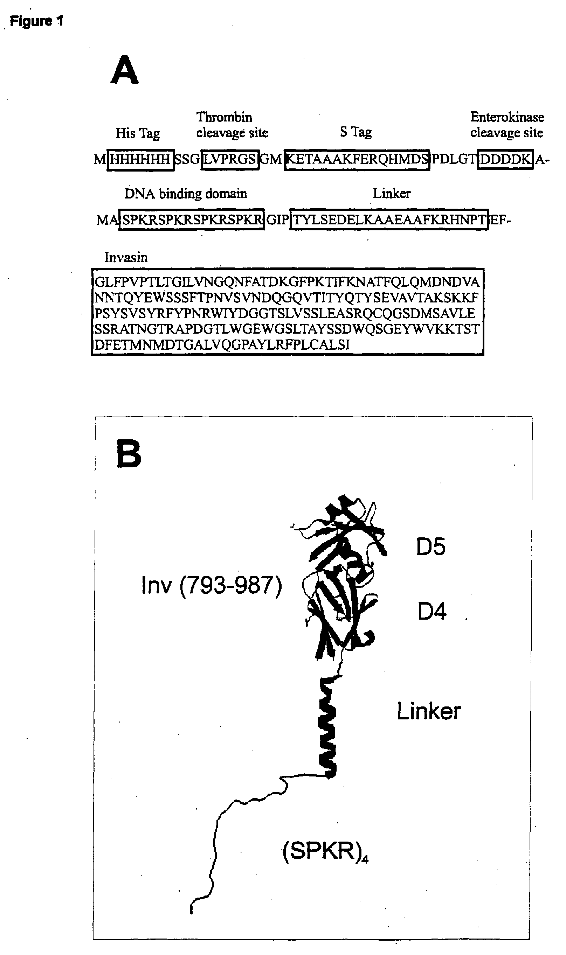

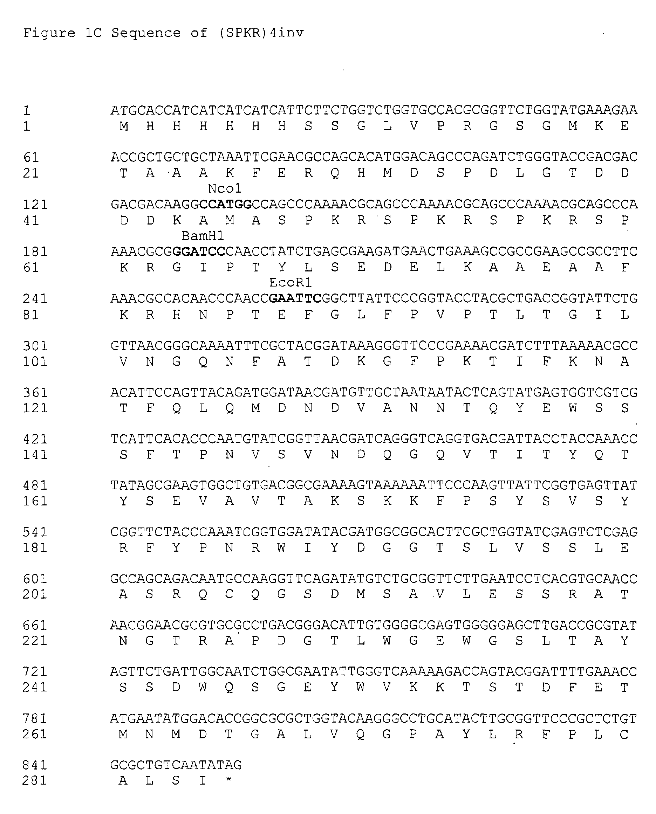

The (SPKR)₄inv protein (Figure 1) contains three functional domains. First, a non-specific DNA-binding domain, (SPKR)₄, which is derived from the histone H1.²⁵ This sequence is able to condense plasmid DNA upon binding to the minor groove. In a previous paper we have shown that a synthetic peptide with this sequence, in combination with a lysogenic peptide, can be used as an efficient transfection agent.²⁶ Second, a rigid a-helical linker domain,²⁷ flanked by flexible proline-glycine sequences, which separates the DNA-binding and receptor-binding elements. This should allow independent action of the two domains. As integrin targeting element of the protein, the C-terminal domain (aa 793-987) of the Invasin protein from *Yersinia pseudotuberculosis* was used. ¹⁹ The DNA fragment encoding amino-acids 793-987 of the *Yersinia pseudotuberculosis* invasin protein, the a-helical linker and the DNA-binding (SPKR)₄ domain, were cloned into the inducible bacterial expression vector pET30a, to obtain the pET30a-(SPKR)₄inv expression vector, as described in the methods section. The complete (SPKR)₄inv protein sequence includes an amino-terminal poly-histidine for Ni-NTA affinity column purification, an S-tag peptide for detection on western blot, and two protease sites for Tag removal (Figure 1). The 22 amino-acid linker is a modification of the sequence published by Gong *et al*.²⁷ An extra N-terminal proline in the linker domain and glycine residues N- and C-terminal to the linker were included, to allow unrestrained movement of the (SPKR)₄ and inv domains. The pET30a-inv construct, which lacks both the linker and DNA-binding domains, was obtained by cloning the invasin sequence as EcoRI/EcoRI fragment into the pET30a vector.

### Bacterial expression of the recombinant proteins.

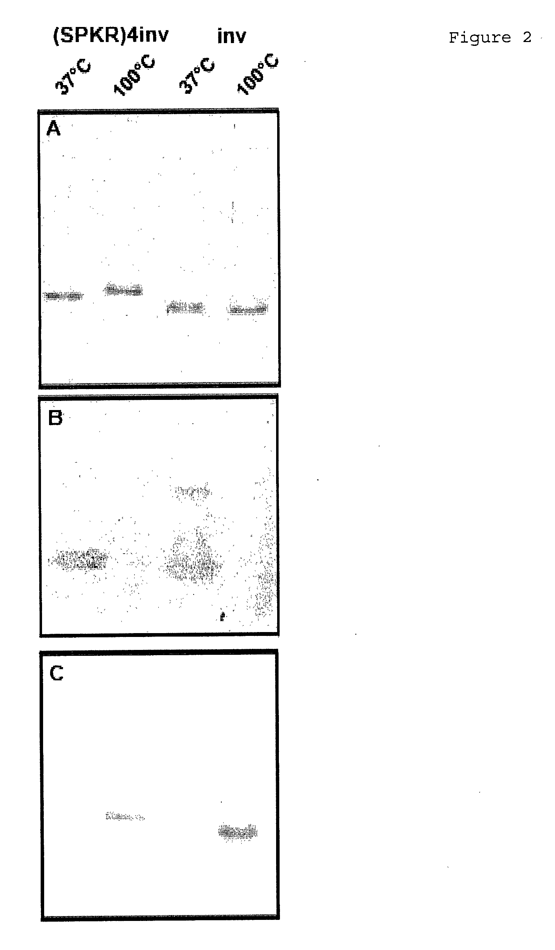

The recombinant proteins were produced in E. coli strain HMS174 (DE3/lysS), which allows tight transcriptional control of the pET30a vector by IPTG induction. At 37 °C, the (SPKR)₄inv and inv proteins accumulated in the induced bacterial cells as insoluble aggregates, at an estimated protein concentration of about 20 mg of protein per liter of bacterial culture. When the bacteria were grown at 20 °C, part of the total protein was produced in soluble form, of which about 0.5 mg could be recovered from a Ni-NTA affinity column per liter culture. After further purification by FPLC chromatography the yield was approximately 100 µg per liter culture. To improve the yield and purity of the produced proteins, another purification method was used, in which insoluble proteins are dissolved and denatured in Guanidin-HCl (see material and methods).²⁴ Purification of (SPKR)₄inv and inv on a Ni-NTA affinity column in the presence of urea yielded 10-15 mg of partially purified proteins per liter bacterial culture (Figure 2). Refolding of the denatured proteins was achieved by stepwise decrease of the urea concentration by dialysis.²⁴ Under these conditions, about half of the protein was recovered in soluble form. After further purification by FPLC chromatography, a yield of 5-7.5 mg protein per liter bacterial culture was obtained. As expected, (SPKR)₄inv and inv have an apparent molecular weight of 33.000 and 32.000 respectively (Figure. 2).

### (SPKR)₄inv binds to plasmid DNA.

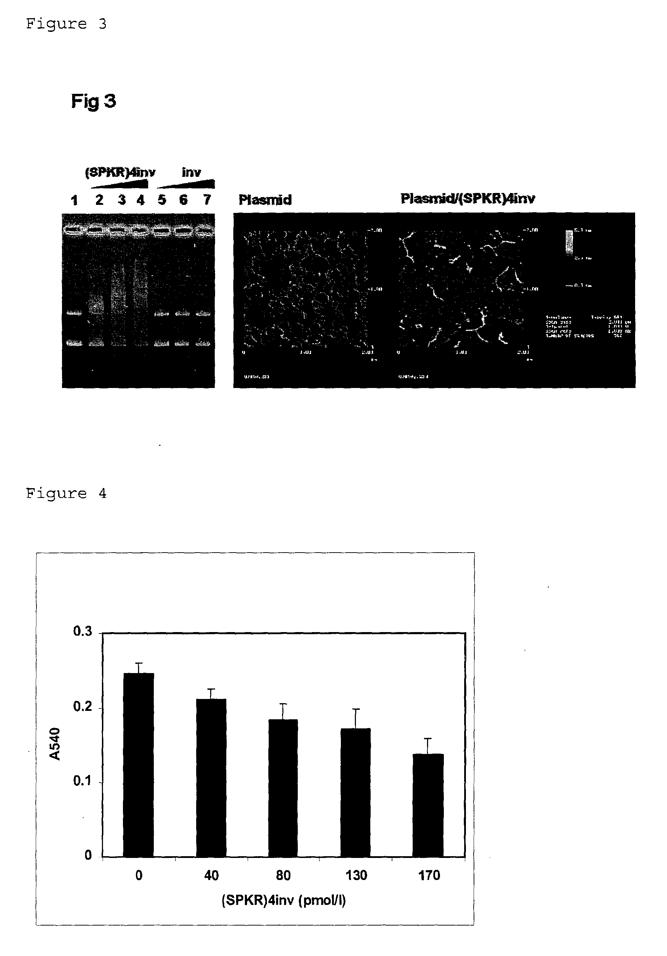

The DNA binding activity of the (SPKR)₄ domain was assessed in a retardation assay. In the presence of (SPKR)₄inv protein, the two plasmid DNA bands (supercoiled and relaxed) showed a different migration pattern at all protein concentrations tested (Figure 3A, lanes 1-4), whereas inv protein at comparable concentrations had no effect on plasmid mobility (Figure 3A, lanes 5-7). Atomic force microscopy revealed that (SPKR)₄inv induces partial condensation of plasmid DNA (Figure 3B). These data show that the DNA-binding domain in (SPKR)₄inv is functional.

### (SPKR)₄inv binds to β1 integrins with high affinity.

The 192 amino-acid C-terminal domain from the *Yersinia pseudotuberculosis* invasin protein was shown to be necessary and sufficient to bind a variety of β1-type integrins on the mammalian cell surface.¹⁹ The ability of the (SPKR)₄inv fusion-protein to bind to cells was tested with a filter overlay assay.²⁸ Proteins were subjected to SDS-PAGE, transferred to a nitrocellulose membrane and exposed to a suspension of HEp-2 cells, which are known to express β1 integrins. The presence of cell-binding proteins on the filter is detected by staining the cells with Amido black. Both (SPKR)₄inv and inv proteins immobilized to a nitrocellulose filter bind to HEp-2 cells (Figure 2B). The protein-cell interaction is only maintained in SDS-PAGE sample buffer at 37 °C but it is lost at 100 °C (Figure 2B). Therefore, the capacity to bind cells is dependent on the proper conformation of the proteins. A temperature-dependent shift of protein mobility was observed in the Coomassie-stained gel, which confirms that such a conformation change occurs (Figure 2A). In addition, the S-tag becomes available for the staining reaction only after boiling the proteins in sample buffer (Figure 2C). These results show the capacity of the native, but not the fully denatured inv-containing recombinant proteins produced in a bacterial expression system to bind integrin expressing cells.

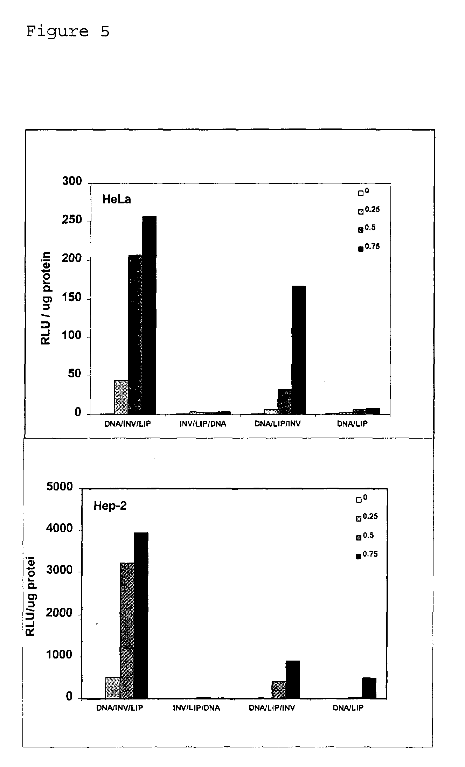

The invasin C-terminus binds to cells by a specific interaction with the β₁ chain of integrins.²⁹ including the α₅ β₁ fibronectin receptor.³⁰ In order to demonstrate that the recombinant (SPKR)₄inv protein binds with high affinity to β₁ integrins, its capacity to interfere with the binding of cells to fibronectin was evaluated in a quantitative attachment assay (see methods section). HEp-2 cells, which express the α₅β₁ integrin, bind efficiently to fibronectin-coated wells, but not to uncoated wells. In contrast, the HT29 cell line, which does not express α₅β₁ integrin,³¹ has a low capacity to bind (Fortunati, unpublished data). Pre-incubation of HEp-2 cells with increasing amounts of (SPKR)₄inv caused a significant inhibition of the binding efficiency (Figure 4). This is evidence for an interaction between the major fibronectin receptor (α₅β₁ integrin) and (SPKR)₄inv, as observed previously for the intact invasin protein.²⁹ As a measure of its binding affinity we calculated the concentration of (SPKR)₄-inv protein that reduces the cell binding to fibronectin coated-wells with 30 % (ID₃₀) (Table 1), from a dose-response curve as shown in Figure 4. The result, 120 nM, is comparable to that obtained with a monoclonal antibody (CD49e) that specifically recognizes the α₅ sub-unit of the human α₅β₁ integrin (Table 1). In contrast, a peptide (RGDS) containing the integrin-binding RGD motif,³² which has been used frequently for integrin targeting of vectors,^{21,22,33} had an ID₃₀ that was three orders of magnitude higher than (SPKR)₄inv. This shows that (SPKR)₄inv binds to its target with much higher affinity than the peptide RGDS. When (SPKR)₄inv was used to coat the wells, the HEp-2 cells bind very efficiently, compared to uncoated wells. For inhibition of cell attachment on (SPKR)₄inv coated wells, more antibody against the α₅ integrin sub-unit (CD49e) was needed than on fibronectin (Table 1). This may be due to the fact that (SPKR)₄inv also interacts with other members of the β₁ integrin family. In strong support of these data, a soluble form of recombinant α₃β₁ integrin³⁴ effectively inhibited the binding of Hep-2 cells to (SPKR)₄inv-coated wells at low concentrations (Table 1), which is further evidence for the specificity of the interaction between (SPKR)₄inv and HEp-2 cells. Much higher concentrations of RGDS peptide were needed to prevent binding of Hep-2 cells to (SPKR)₄inv (Table 1). In summary, these data show that the inv-containing fusion-proteins are active in binding to β1 integrins, in particular α₅β₁ and α₃β₁, with an affinity that is comparable to a monoclonal antibody, and much higher than an RGD peptide. The results shown here were achieved with proteins made with the renaturation method. Proteins isolated from the soluble fraction gave comparable results (not shown).

### (SPKR)₄inv increases the transfection efficiency of a lysogenic compound.

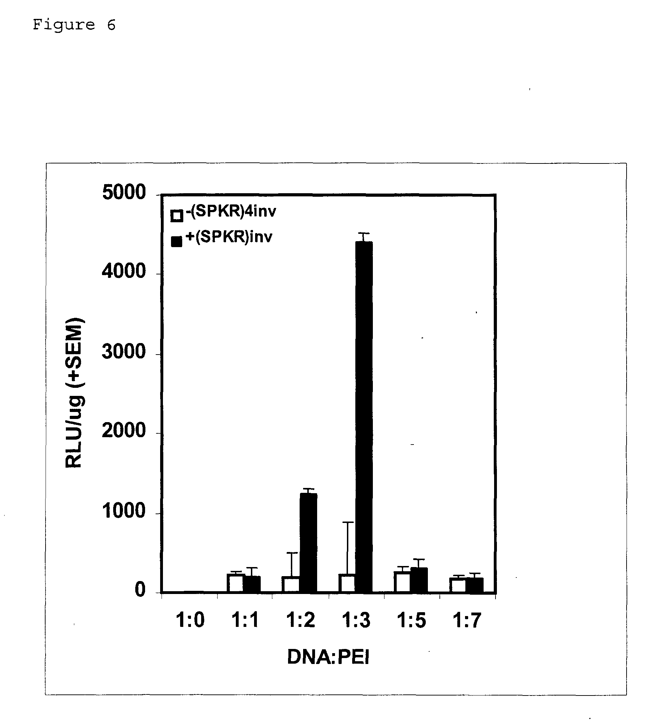

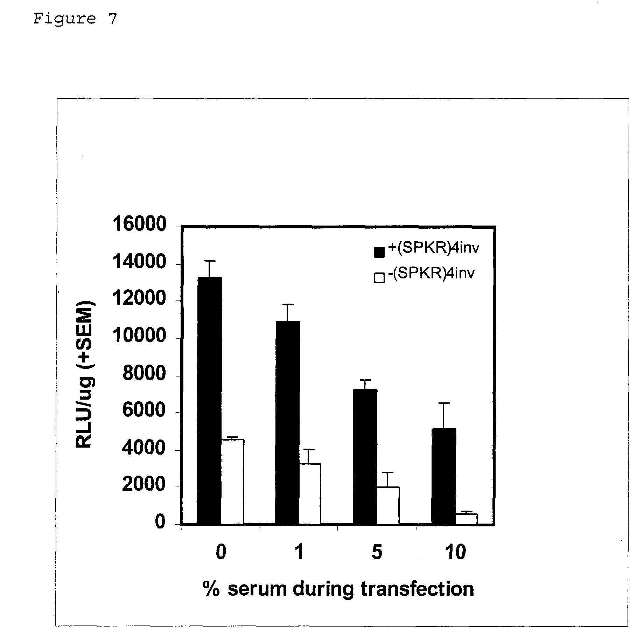

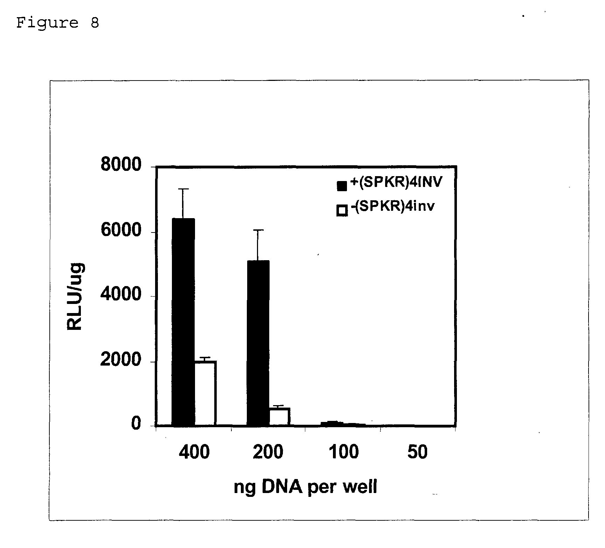

(SPKR)₄inv complexes with fluorescent plasmid DNA, form aggregates in culture medium of 300-500 nm, which are detected on the cell surface of the HEp-2 cells, immediately after treatment. This is not observed with fluorescent plasmid DNA alone (not shown). This shows that the recombinant (SPKR)₄inv protein is able to target DNA to the cell surface. However, (SPKR)₄inv -plasmid complexes do not efficiently transfect cells *in vitro* (Figure 5). This is not surprising, since it is known that escape from the lysosomal compartment is an essential requirement, and (SPKR)₄inv-DNA complexes do not have such a capacity. Therefore, the cationic DOTAP liposome was tested as helper agent. When DOTAP was added to (SPKR)₄inv/DNA complexes that were solubilized in a binding buffer containing 0.1% Tween-20 (see methods), considerably higher luciferase expression was obtained in both HeLa and Hep-2 cells (Figure 5, DNA/(SPKR)₄inv/LIP) than with DOTAP alone (Figure 5, DNA/lip). Under these conditions, DOTAP forms cationic mixed micelles with Tween-20 that are apparently not effective in transfection. Varying the (SPKR)₄inv concentration showed that optimal activity was obtained at a 1:1 DNA/protein weight ratio (not shown). If DOTAP and (SPKR)₄inv were mixed before addition of plasmid, virtually no expression was observed (Figure 5, INV/LIP/DNA). Addition of (SPKR)₄inv to DNA/DOTAP complexes (Figure 5, DNA/LIP/INV) gave intermediate results. These data show that (SPKR)₄inv, in combination with a lysogenic carrier, promotes transfection efficiency *in vitro.*
In another series of experiments, the cationic polymer polyethylenimine (PEI), which is a simple and effective transfection agent⁵⁻⁷ was used as a lysogenic and DNA condensing helper substance. Plasmid DNA, complexed to (SPKR)₄inv and control, was mixed with different amounts of PEI, and the complexes were used to transfect HeLa cells (Figure 6). The data show that DNA/(SPKR)₄inv/PEI complexes consistently give a considerably (4-10 fold) higher marker gene expression than DNA/PEI alone. These data were confirmed in four similar, independent titrations. The loss of activity at high PEI/DNA ratios (Figure 6) could be attributed to toxicity of the PEI, as shown by a strong reduction of the protein recovery (data not shown). Variation of the amount of (SPKR)₄inv resulted in an optimal weight ratio under these conditions (DNA: protein: PEI) for maximal luciferase expression of 1:1:3 (not shown). Addition of fetal calf serum during transfection did reduce the markergene expression of both PEI and (SPKR)₄inv/PEI complexes, but not the stimulatory effect of (SPKR)₄inv (Figure 7). The difference between DNA/(SPKR)₄inv/PEI and DNA/PEI complexes was actually greatest at high (10%) serum concentrations. The total luciferase activity obtained depended strongly and in a nonlinear way on the amount of (SPKR)₄inv/PEI and PEI complexes added per well (Figure 8).

### The recombinant protein-mediated gene transfer is integrin-specific.

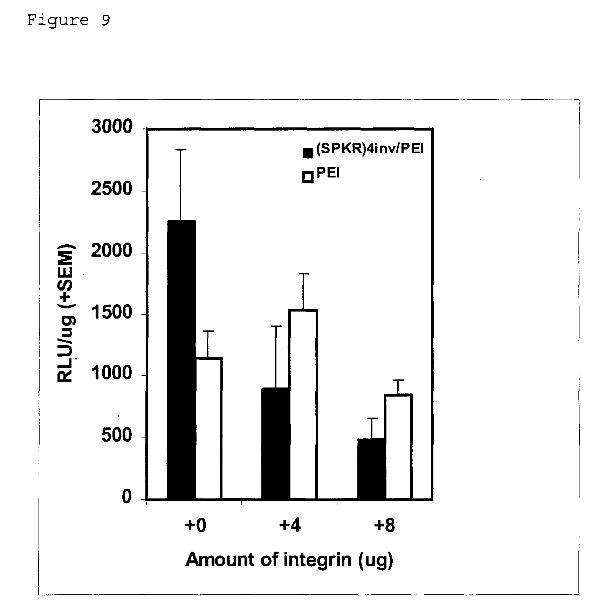

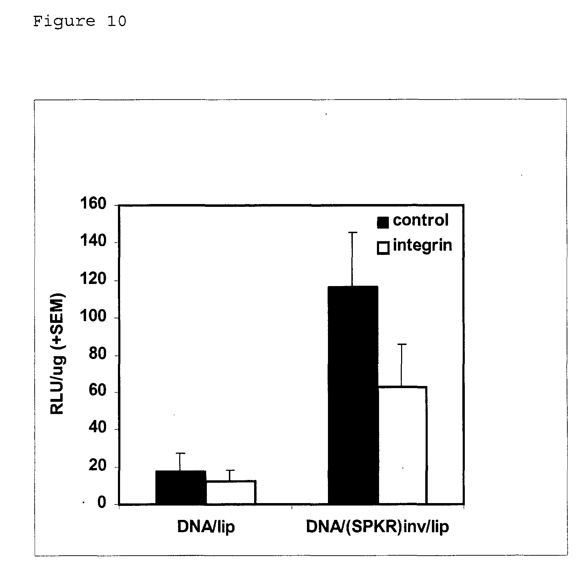

The target specificity of (SPKR)₄inv mediated gene transfer was investigated using soluble α₃β₁ recombinant integrin as a competitor. Pre-incubation of a DNA/(SPKR)₄inv /PEI transfection mixture with different amounts of recombinant α₃ β₁ for 30 min at room temperature, resulted in a marked reduction in marker gene expression after transfection of HeLa cells (Figure 9). In contrast, the efficiency of the DNA/PEI preparation was not significantly affected. These data support the conclusion that (SPKR)₄inv is capable to target DNA specifically to β₁ integrins, in the presence of PEI. A similar result was obtained with DNA/(SPKR)₄inv/DOTAP complexes (Figure 10).

### Discussion

We set out to investigate whether it is feasible to target plasmid DNA complexes to a cellular receptor with a multi-domain protein that contains a non-specific DNA-binding protein. This approach allows us to introduce a receptor binding element to transfection complexes without the need of chemical coupling, which is generally a tedious and inefficient procedure leading to random products. In addition, it enables us to use a mixture of ligands in a simple way. This greatly increase the selectivity for a cell type that exposes a particular combination of receptors. For this purpose, the (SPKR)₄inv fusion-protein was designed to have two independent functional domains, separated by a rigid α-helical linker (Figure 1). One domain is a non-specific DNA binding sequence (SPKR)₄, derived from an H1 histone that is involved in sperm chromatin condensation.²⁵ The other element is the amino-terminal domain from the *Yersinia pseudotuberculosis* invasin protein, which is known to bind β1 integrins with high affinity.^{19,20,35,36} The (SPKR)₄inv protein and the inv protein, which lacks the DNA binding domain, were produced in a commercially available bacterial expression system. Both proteins could be recovered at high yield from the denatured bacterial lysate after renaturation by controlled dialysis, and purified in an active form by Ni-affinity and FPLC column chromatography (Figure 2). Retardation experiments and atomic force microscopy confirmed the expected binding affinity of (SPKR)₄inv for plasmid DNA, in contrast to the Inv protein (Figure 3). Cells that express β1 integrins bind to (SPKR)₄inv and inv in a blot overlay assay (Figure 2). This property is lost when the proteins are completely denatured in SDS (Figure 2), showing that the capacity of the recombinant proteins to interact with cells is completely dependent on their correct conformation, as described previously for the complete invasin protein.¹⁹ We were further able to show that the recombinant invasin proteins bind specifically and with high affinity to β1 integrins using a quantitative cell attachment assay (Figure 4, Table 1). A monoclonal antibody (CD49e) against the α5 sub-unit of the major α5β1 fibronectin receptor interfered effectively with cell binding to a fibronectin coated surface. (SPKR)₄inv had the same effect, when molar concentrations in the same range were used (Table 1). Similarly, binding of cells to a (SPKR)₄inv coated surface was inhibited by the CD49e antibody. Most significantly, a soluble form of α3β1 integrin³⁴ inhibited cell binding to (SPKR)₄inv at nanomolar concentrations (Table 1). These data show that the renatured multidomain protein (SPKR)₄inv interacts at high affinity with β1 integrins as was demonstrated previously for invasin.^{19,20,36} The apparent affinity of (SPKR)₄inv for its target receptor in this assay was three orders of magnitude higher than that of a commercially available small peptide ligand of β1 integrins (RGDS). This is an important advantage of the invasin domain in β1 integrin targeted compounds, compared to the synthetic peptide ligands. The S-tag (Figure 1), is apparently not exposed in native (SPKR)₄inv and inv, since it is only detected on western blot when the samples are completely denatured (Figure 2). This suggests that the tag is involved in an intra-molecular interaction that prevents binding of the probe. This could explain why it proved difficult to remove the tag from the native proteins by treatment with enterokinase (data not shown). Since the Tag does not interfere with either the DNA- or integrin-binding capacity of the proteins, the complete proteins were used in the experiments presented here.

To demonstrate that (SPKR)₄inv can be used as a targeting device in combination with a DNA condensing and lysogenic carrier, a series of transfection experiments was performed with cells known to express β1 integrins. We observed that (SPKR)₄inv alone was unable to transfect cells, but significantly increased transfection efficiency of both DOTAP and PEI complexes (Figure 5, 6). As expected, the DNA/protein ratio that was found to be effective in transfection experiments (Figure 5, 6) does not result in complete plasmid DNA retardation or condensation (Figure 3). This is evidence for a residual negative surface charge of the complexes, which would allow binding of a cationic carrier such as DOTAP of PEI. This, in turn, is required to allow efficient escape of the DNA from the lysosomal compartment.
Pre-incubation of (SPKR)₄inv containing DOTAP and PEI complexes with a soluble form of α₃β₁ integrin significantly reduced their transfection efficiency (Figure 9, 10). This shows that transfection with (SPKR)₄inv containing compounds is indeed integrin mediated under these conditions. Increasing the transfection efficiency of the receptor-mediated DNA complexes by addition of a cationic lipid has been shown previously.^{21,37} The lipid is likely to be involved both in DNA condensation and in the escape of the complexes from the lysosomal compartment. Optimal conditions for selective integrin directed transfection involves an amount of liposome per DNA that is insufficient by itself, but effective in the presence of integrin-targeted protein (Figure 5). During our titration experiments we were able to create such conditions using mixed micelles of DOTAP and Tween-20, a non-ionic detergent with a low critical micelle concentration. Similarly, a mixed complex of plasmid DNA, (SPKR)₄inv and the cationic polymer PEI proved to be more effective in transfection than DNA/PEI complexes that were made under the same conditions (Figure 6). The role of PEI in the mixed complexes is expected to be comparable to that of DOTAP, i.e., DNA condensation and promoting escape from the lysosomal compartment. Addition of fetal calf serum in the transfection medium reduced the marker gene expression but did not abolish the stimulatory effect of (SPKR)₄inv (Figure 7). This indicates that such a formulation could be effective *in vivo.* However, interaction with serum components is not the only issue. Titration of the amount of PEI showed an optimum (Figure 6), suggesting that a minimal amount of PEI was required to obtain efficient transfection, whereas higher amounts proved toxic to the cells. In addition, one of the most important determinants of transfection efficiency in the titration experiments proved to be the amount of complexed DNA per cell (Figure 8). The apparent non-linearity of this relationship also suggests that deposition of a minimal amount of lysogenic carrier per cell is required to allow efficient escape from the endosomal compartment. This should be kept in mind when developing targeted gene transfer systems for use *in vivo.* Selective uptake is only useful when combined with efficient endosomal escape. On the other hand, the lysogenic capacity of the carrier should not lead to toxicity. Furthermore, the cationic carrier is likely to contribute significantly to non-specific uptake of complexes by the cells, and it should therefore be minimized. It is thought that much of the success *in vitro,* and lack of efficiency *in vivo,* of conventional cationic carrier systems depends on the positive surface charge of the complexes. This results in binding to the negatively charged plasma membrane promoting uptake, but also reduces the biological halftime and delivery to target cells of the complex.⁹ Reduction of the surface charge to zero or negative values would solve this problem. This can be achieved by shielding of the surface charge by coating the complexes with polyethylenglycol (PEG).⁹ Therefore, further improvements of the integrin targeted delivery system described in this paper can be expected from the development of novel PEGylated carriers that allow efficient endosomal escape while reducing nonspecific interactions.
In conclusion, the exemplified gene delivery vehicle of the present invention, (SPKR)₄inv is a novel multi-domain protein that binds both DNA and β1 integrins. In combination with a lysogenic poly-cation, (SPKR)₄inv is able to transfect cells *in vitro* in a β1-integrin dependent way. β1 integrin targeting ligands have been used successfully *in vitro,* when coupled chemically to a cationic polymer.^{22,33} However, we show here that (SPKR)₄inv binds to β1 integrins with a much higher affinity than the available RGD-type peptide ligands. In addition, the DNA binding domain of the protein makes inefficient and tedious coupling procedures unnecessary. Moreover, the modular design of the pET30a (SPKR)₄inv expression vector allows the introduction of an alternative ligand by a simple cloning procedure. Another advantage of this chimaeric protein is that it will bind to any DNA expression vector, in contrast to a similar protein with a specific (Gal4) DNA-binding domain.²⁴ As pointed out in the introduction, integrins offer possibilities for *in vivo* targeting of damaged or inflamed tissues,^{12,15-17} or tumors.¹⁴

### Materials and methods

### Cell lines

A human epidermal carcinoma cell line (HEp-2), was obtained from American Type Culture Collection (ATCC) at passage number 364. Human epithelial cervix- (HeLa) was kindly provided by A. Raams (Erasmus University, Rotterdam, The Netherlands). All cell lines were cultured in 1:1 (v:v) DMEM:F10⁺ supplemented with 10% fetal calf serum, 2 mM glutamine, 50 U/ml penicillin, and 50 µg/ ml streptomycin.

### Cloning and expression vectors

The (SPKR)₄ DNA-binding sequence derived from the H1B histone^{25,26} was inserted into the pET-30a bacterial expression vector (Novagen inc., Madison, Wi), digested with Nco1 and BamH1, followed by ligation of annealed double-stranded oligonucleotides (5'-CATGGCC(AGCCCAAAACGC)4GG-3', 5'-GATCCC(GCGTTTTGGGCT)₄GGC-3'). This results in an in-frame fusion of the DNA binding motif to 6 repeated histidine residues (His-tag) and a (S-tag) motif, which allow easier purification and a fast identification of the produced recombinant protein, respectively (Figure 1). Next, a 71 bp double-stranded oligonucleotide, encoding an α-helix peptide linker,²⁷ was ligated as BamHI/EcoRI fragment into the pET30a-SPKR plasmid. The plasmid was sequenced and no mutations were found.
The DNA sequence of the invasin gene corresponding to amino acids 793-987 of the published sequence,¹⁹ was amplified by PCR from *Yersinia pseudotuberculosis* chromosomal DNA (RIVM, Bilthoven, The Netherlands). The primers (Eurogentec, Seraing, Belgium) used for the PCR reaction were 5'-ATTCCCGGTACCTACGC-3' and 5' -GAATGCTATATTGACAGCG-3'. The PCR fragment was cloned first into an AT-vector (Dr. B. Beverloo, Erasmus University, Rotterdam), and then transferred as an EcoRI fragment into the pET30a-SPKR-linker vector, 3' to the linker sequence. The resulting plasmid (pET30a-(SPKR)₄inv), encodes the (SPKR)₄inv protein (Figure 1). The last cloning step resulted in addition of four extra amino-acids (EFGL), 3' to the linker sequence (Figure 1). DNA sequencing identified 7 point mutations in the invasin domain compared to a sequence previously published.¹⁹ Four were silent mutations and the others resulted in amino-acid changes: R→T (position 895), Q→V (position 971), P→Q (position 972).
None of these mutations affect sequences known to be critical for integrin-binding,¹⁹ which is confirmed by our data (Table 1, Figure 2, Figure 4). The plasmid pET30a-inv, encoding the invasin domain aa 793-987, which lacks the DNA-binding and linker domains, but includes the Tag sequence was obtained by ligation of the EcoRI invasin domain fragment into the pET30a vector. The invasin domain sequence of the resulting plasmid was identical to the one in pET30a-(SPKR)₄inv.

### Bacterial expression and purification of the recombinant proteins.

First method: production of soluble proteins: Log-phase cultures of *E*. *coli,* strain HMS174(DE3/LysS) carrying the plasmid pET30a-(SPKR)₄inv, or pET30a-inv were grown to OD₆₀₀ of 0.9 at 20 °C in Luria-Bertani medium supplemented with 30 µg/ml kanamycin (Gibco) and 34 µg/ml chloramphenicol (Fluka). The expression of the recombinant proteins was induced by the addition of 1 mM isopropyl-β-D-thiogalactopyranoside (IPTG, Sigma-Aldrich). The cells were harvested by centrifugation three hours after IPTG induction, and stored at -20 °C. The frozen pellet was resuspended in buffer A: 20 mM Tris-HCl pH 7.9, 1M NaCl, 2 mM Imidazole, 10% glycerol, 0.1% Tween-20 and 0.1 mM phenyl-methyl-sulphonyl-fluoride (PMSF, Sigma-Aldrich), and lysed by sonication on ice. The cell lysate was cleared by centrifugation (10.000xg 30 min at 4 °C) and loaded onto Ni-NTA resin (Quiagen) which was previously equilibrated in buffer A. The column was washed with ten column volumes of buffer B (20 mM Tris-HCl pH 7.9, 0.5 M NaCl, 20 mM Imidazole 10% glycerol, 0.1% Tween-20 and 0.1 mM PMSF). Recombinant proteins were eluted with a 10 column volume linear gradient of imidazole (20-600 mM) in buffer B. The eluted fractions were analyzed by SDS gel-electrophoresis, the recombinant proteins were identified on western blot using the S-tag system (Novagen, R&D Systems Europe Ltd, Abingdon, UK). The method is based on the specific binding between the S-protein, conjugated to alkaline phosphatase, and the 15 amino-acid S-tag peptide of the recombinant proteins. The fractions containing recombinant proteins were pooled and dialyzed to buffer C (20 mM Tris, 100 mM NaCl, 10% glycerol, 0.1% Tween 20, pH 7.9), further purified using an FPLC liquid chromatography device (Pharmacia, Uppsala, Sweden), on a MonoQ (inv) or MonoS column (SPKR₄-inv) equilibrated with Buffer C and eluted with a linear NaCl gradient. In both cases the proteins eluted at about 250 mM NaCl.
Second method: purification of denatured recombinant proteins and renaturation by dialysis. The procedure followed is as published by Peul *et al*.,²⁴ with minor modifications. The transformed bacteria were grown at 37°C, and harvested three hours after induction, as described above. The frozen bacterial pellet of 0.5 l culture was dissolved in 20 ml 6 M Guanidin-HCl, 0.5 M NaCl, 20 mM NaPO4, pH 7.8, sonicated, and centrifuged to clarity (10.000 g, 10 min). The supernatant was loaded on a 5 ml Ni-NTA affinity column equilibrated with buffer D: 6 M Urea, 0.5 M NaCl, 20 mM NaPO4, pH 7.8. The column was washed with ten volumes of buffer D. The recombinant proteins were eluted with a pH gradient (7.8-3.5) in buffer D. Fractions containing recombinant proteins were dialyzed overnight against buffer E (6 M urea, 20 mM Tris-HCl pH 7.5, 0.1 M NaCl, 10% glycerol, 5 mM β-mercaptoethanol, 0.1% Tween-20). Renaturation of the proteins was achieved by stepwise removal of urea by dialysis. Eight two-fold dilutions of the dialysis buffer with urea-free buffer E were performed at two hour intervals, at 4°C. Tween-20 was added during the renaturation procedure to prevent precipitation due to hydrophobic interactions.²⁴ However, we have found that functional (SPKR)₄inv could be renatured with the same efficiency in the absence of Tween-20. After removing precipitates by centrifugation, the proteins were purified on a mono-Q (inv) or mono-S ((SPKR)₄inv), FPLC column. Peak-fractions were concentrated to 0.2 g/l with a Centricon-10 centrifugal concentrator (Amicon, Beverly, Ma, USA).

### DNA-retardation assay

0.25 µg of plasmid DNA (pCMVGFP) was incubated with increasing amounts of SPKR₄inv protein per 20 µl DNA binding buffer (10 mM Tris-HCl pH 7.5, 100 mM NaCl, 10% glycerol, 0.01% Tween-20) for 30 min at room temperature. The samples were analyzed on a 1% agarose gel in Tris-Acetate-EDTA (TAE), stained with ethidium bromide.

### Atomic Force Microscopy

DNA samples and DNA protein complexes were prepared in binding buffer as described above, and subsequently diluted 10 fold in 5 mM HEPES, 2 mM MgCl2, pH 7.4. 20 µl samples were deposited on mica. After 1 min the mica was washed with HPLC grade water (Sigma-Aldrich), and dried in a stream of filtered air. Images were acquired on a Nanoscope IIIa (Digital instruments Inc, Santa Barbara, Ca) operating in tapping mode in air, using Nanoprobe Silicon tips (Digital instruments Inc, Santa Barbara, Ca).

### Cell binding to filter-immobilized recombinant proteins

The assay was performed according to Leong *et al*.,²⁸ with minor modifications. The samples containing the recombinant proteins were loaded on a 11% SDS-PAGE gel and run at low voltage to avoid heating of the samples. Proteins were subsequently transferred to nitrocellulose filters (Ba85, Schleicher & Schuell, Dassel, D) by Western blotting. To avoid non-specific protein binding, filters were preincubated overnight at 4 °C in phosphate buffered saline plus 10 g/l bovine serum albumin (BSA). HEp-2 cells were trypsinized, washed in medium containing 1mg/ml soybean trypsin inhibitor (type I-S, Sigma-Aldrich) and resuspended in medium supplemented with 20 mM HEPES pH 7.4 and 4 g/l BSA, at a concentration of 10⁶ cells/ml. The filters were incubated with 10 ml of this cell suspension at 37 °C for 1 hour. After washing three times in PBS, the filters were fixed with 3% (w/w) para-formaldehyde in PBS for 10 min at 20 °C, stained with 1% Amido-black in 40% methanol, 10% acetic acid, and destained with 40% methanol, 10% acetic acid.

### Cell attachment to coated wells.

Cells were harvested with trypsin-EDTA and resuspended in medium containing soybean trypsin inhibitor. After centrifugation, the cell pellet was resuspended in medium containing 20 mM HEPES pH 7.5, 4 g/l BSA. For inhibition studies, 3x10⁴ cells were incubated with increasing concentrations of SPKR₄-inv protein for 30 min at 22 °C. Alternatively, RGDS peptide (Gibco, Breda, NL), or the monoclonal anti-α₅β₁ CD49e antibody (Serotec.Ltd, Oxford, UK) and the recombinant soluble α₃β₁ were used.³⁴ The treated cells were transferred to a 96-well plate coated with 12 µ g/ml fibronectin from human serum (Sigma-Aldrich) dissolved in PBS or with 2 µg/ml invasin as described by Van Nhieu *et al*.²⁹ After incubation for 1 hour at 37 °C, the cells were washed twice with PBS and quantified by the crystal violet staining method.³⁸ The concentration that results in 30% inhibition (ID₃₀) was calculated from a dose-response curve, and used as a measure of affinity.

### Transfection mixtures and measurements of the luciferase expression.

For transfection experiments, HeLa or Hep-2 cells were seeded at a density of 2-3X10⁴ cells per well in 24-well plates, transfection was performed the following day. Plasmid-protein-DOTAP complexes were prepared as follows: per well, 0.25 µg of pCMVluc was mixed with variable amounts (0-1 µg) of (SPKR)₄inv protein in 10 µl, 10 mM Tris-HCl pH 7.5, 100 mM NaCl, 10% glycerol, 0.1% Tween-20. After 30 minutes at room temperature, different amounts (0-2 µg) of DOTAP liposomes (Boehringer-Mannheim, Germany), diluted in HEPES buffered saline, were added. The complexes were left at room temperature for a further 30 min before being diluted in 0.2 ml medium without serum and antibiotics, and added to the cells. After 3-4 hours incubation, the mixture was replaced by culture medium containing 10 % fetal calf serum. After 35-48h incubation, cells were lysed with 70 µl lysis buffer (25 mM Tris-phosphate, pH 7.8, 10 g/l Triton-X 100, 1 mM DTT, 15% (v/v) glycerol) for 20 min at room temperature. 10 µl of supernatant was assayed for luciferase activity as described. Luciferase activity was expressed as relative light unit (RLU) per µg of protein in the cell lysates. Protein concentration was determined with the BCA Protein assay (Pierce, Rockford, Illinois, USA).

Transfections of HeLa cells with Polyethylenimine complexes were performed essentially as described above. All transfection experiments performed with polyethylenimine shown in this paper were performed in the absence of Tween-20. Titration with complexes made in the presence of Tween-20 resulted in comparable results (data not shown). Polyethylenimine (PEI, Aldrich, mw 23.000), extensively dialyzed against water and neutralized to pH 7.5, was diluted in phosphate buffered saline (PBS). Plasmid DNA was diluted to 1 µg per 80 µl 10 mM Tris-HCl pH 7.5, 100 mM NaCl, 10% glycerol, to which the appropriate amount of (SPKR)₄inv solubilized in the same buffer was added. After 30 minutes incubation at room temperature, an equal volume of PEI (0-3 µ g) in PBS was added to the concentration indicated. After a further 30 minutes incubation, the complexes were diluted in 800 µl pre-warmed serum free medium (DMEM/F10) and added to the cells (200 µl per well, 3X10⁴ cells). After 3-4 hrs, the mixture was replaced by culture medium containing 10 % fetal calf serum. After 35-48h incubation, cells were lysed and luciferase activity was determined as described above.

### Brief description of the drawings

Figure 1. Protein sequence and structure of (SPKR)₄inv.
   A: Protein sequence of the recombinant (SPKR)₄inv protein. The protein domains were cloned into the bacterial expression vector pET30 as described in the methods section. The Inv protein lacks both the linker and DNA binding domain.
   B: A three-dimensional model of (SPKR)₄inv, without the tag sequence, based on a recently published model of invasin [Hamburger, 1999] and a computer prediction of the (SPKR)4-linker domain. D4 and D5 together constitute the integrin binding domain of invasin, the rigid a-helical linker connects the DNA binding domain (SPKR)₄ to the inv domain through flexible glycine and proline containing sequences (Figure la: ..SPKR**GIP**TY.. and ...RHN**PTEFG**LF.... respectively).
   C: sequence of reading frame
Figure 2. Gel electrophoresis, overlay blotting and western blotting of recombinant proteins. A: SDS-polyacrylamide gel electrophoresis analysis (top of the gel to front) of the recombinant (SPKR)₄inv and inv proteins after FPLC purification, Coomassie stain. The apparent molecular weight of the proteins (33.000 for (SPKR)₄inv, and 32.000 for inv) was determined relative to a set of low molecular weight standards (not shown, Pharmacia). Proteins were solubilized in sample buffer and incubated at either 37 °C or 100 °C for five minutes. B: Cell-binding activity of the proteins was determined by overlay blotting as described. The data show that HEp-2 cells only bind to native (37°C) (SPKR)₄inv and inv. C: Western-blot. The proteins immobilized on the filter were tested for binding to phosphatase conjugated S-protein (Novagen, Madison Wi, USA). The data show that the S-tag is only exposed after complete denaturation of the proteins (100°C).
Figure 3. (SPKR)₄inv, but not inv, binds to plasmid DNA. A: gel retardation assay. pCMVGFP plasmid DNA (0.25 µg, lane 1) was mixed with increasing amounts (0.5, 1, 2 µg) of (SPKR)₄-inv (lanes 2,3,4) or inv protein (lanes 5,6,7) as described in the methods section. After 30 min at room temperature the samples were loaded on a 1% agarose gel. B: Atomic force microscopic images (see methods section) of supercoiled plasmid DNA (pCMVGFP, left panel) and plasmid-(SPKR)₄inv complexes (1:1 w:w, right panel). Both images were collected as 4 µm² fields, color indicates height as indicated by the z-range bar.
Figure 4. (SPKR)₄inv interferes with cell binding to fibronectin. The attachment of HEp-2 cells in suspension to fibronectin coated-wells is reduced by pre-incubation with increasing concentrations of (SPKR)₄inv. The assay was performed as described in the methods section. Attachment of cells was measured as the absorbance at 540 nm (A540) after crystal violet staining. The bars represent the mean of 4 values, the error bars indicate the standard deviation. Uncoated wells result in an A540 of 0.0 under these conditions.
Figure 5. Transfection efficiency of plasmid-(SPKR)₄inv complexes in the presence of DOTAP. HeLa (upper panel) and HEp-2 (lower panel) cells were transfected with a Luciferase expression vector (pCMVLuc), as described in the methods section. The transfection mixtures were prepared, by mixing in three different orders (as indicated) : 0.25 µg pCMVluc (DNA), 0.5 µg (SPKR)₄inv (INV), and varying amounts of the cationic lipid DOTAP (LIP, 0-0.75 µg), as described in the methods section. DNA/LIP indicates that (SPKR)₄inv was replaced by buffer alone, to assess the efficiency of DOTAP under these conditions. 24 hours after seeding, the cells were incubated for 4 hours with the transfection complexes diluted in serum-free medium. Luciferase gene expression was measured in cell lysates 1 day after transfection, as described. The bars represent the average of two independent luciferase measurements, expressed as relative light units (RLU) per µg protein. Similar results were obtained in three independent titrations.
Figure 6. (SPKR)₄inv increases the transfection efficiency of PEI in HeLa cells. Plasmid/(SPKR)₄inv complexes (1:1, w:w) were prepared as described in the methods section. Different amounts of PEI were added to the DNA-protein complexes (black bars), as indicated (DNA:PEI, w/w). As a control, (SPKR)₄inv was replaced by buffer (open bars). The complexes (1 µg DNA per condition) were diluted in serum-free medium and divided over four wells of a 24-well plate, each containing 3.10⁴ HeLa cells, 24 hours after seeding. After 4 hours, the medium was replaced with serum-supplemented medium. Luciferase activity was determined 48 hours after transfection. Bars represent averages of four values, error bars indicate standard error of the mean.
Figure 7. The effect of serum during transfection of HeLa cells with DNA/(SPKR)₄inv/PEI complexes. HeLa cells were transfected with DNA/(SPKR)₄inv/PEI complexes (1:1:2.5, w:w, black bars), or DNA/PEI complexes (1:2.5. open bars) as control. The experiment was performed as described in the legend of Figure 6, except that the complexes were diluted in medium with different amounts of fetal calf serum, as indicated. Bars represent averages of four values, error bars indicate standard error of the mean.
Figure 8. Marker gene expression Transfection of HeLa cells with different amounts (ng DNA per well, containing 3.10⁴ cells) of DNA/(SPKR)₄inv/PEI (1:1:3, w:w, Black bars), or DNA/PEI (1:3, w:w, open bars). The transfection experiment was performed as described in the legend of Figure 6. Bars represent averages of four values, error bars indicate standard error of the mean.
Figure 9. Soluble integrin (α₃β₁) inhibits the transfection efficiency of (SPKR)₄inv mediated transfection. HeLa cells were transfected with DNA/(SPKR)₄inv/PEI complexes (1:1:3, black bars), or DNA/PEI complexes (1:3, open bars) as described in the legend of Figure 6, except that the complexes were incubated for 30 minutes with different amounts of soluble α₃β₁ integrin [Eble, 1998], as indicated ( µg α₃β₁ per µg DNA), before dilution in medium. Bars represent averages of four values, error bars indicate standard error of the mean.
Figure 10. (SPKR)₄inv mediated gene transfer is integrin-dependent. HEp-2 cells were transfected with DNA/(SPKR)₄inv/DOTAP complexes (0.25µg/ 0.5µg/ 0.25µg) or DNA/DOTAP, as described in the methods section (Solid bars). In a parallel experiment, part of the complexes were incubated with 2.5 µg of recombinant α₃β₁ integrin for 30 min at room temperature (open bars). Expression of the markergene luciferase was evaluated after 24 hours. The bars represent the average of three independent values, error-bars indicate standard deviation.

**Table 1**

| Competitive inhibition of HEp-2 cell attachment to coated wells. | | |
|---|---|---|
| ID₃₀ (µmol/l) | | |

| | Wells coated with | |
|---|---|---|
| Inhibitors | Fibronectin | (SPKR)4inv |
| (SPKR)₄inv | 0.120 | ND |
| CD49e | 0.020 | 0.186 |
| RGDS | 170 | 170 |
| α₃β₁ | ND | 0.050 |

Competitive inhibition of HEp-2 cell attachment to fibronectin- or (SPKR)₄inv coated wells, was determined as described in the methods section. The table shows the concentration of competitor that results in 30 % inhibition of cell attachment (ID₃₀). The reported values are calculated from a dose-response curve in cell attachment experiments as shown in Figure 4. Each dose was tested in quadruplicate. The obtained ID₃₀ values each showed a standard deviation of about 20% of the mean.
N.D.: not determined

### References

1. Batra RK *et al*. Receptor-mediated gene delivery employing lectin-binding specificity. *Gene Ther* 1994; **1:** 255-60.
2. Ziady AG *et al.* Ligand substitution of receptor targeted DNA complexes affects gene transfer into hepatoma cells. *Gene Ther* 1998; **5:** 1685-97.
3. Hoganson DK *et al.* Targeted delivery of DNA encoding cytotoxic proteins through high- affinity fibroblast growth factor receptors. *Hum Gene Ther* 1998; **9:** 2565-75.
4. Perales JC *et al.* Gene transfer in vivo: sustained expression and regulation of genes introduced into the liver by receptor-targeted uptake. *Proc Natl Acad Sci U S A* 1994; **91:** 4086-90.
5. Boussif O *et al.* A versatile vector for gene and oligonucleotide transfer into cells in culture and in vivo: polyethylenimine. *Proc Natl Acad Sci U S A* 1995; **92:** 7297-301.
6. Baker A *et al.* Polyethylenimine (PEI) is a simple, inexpensive and effective reagent for condensing and linking plasmid DNA to adenovirus for gene delivery. *Gene Ther* 1997; **4:** 773-82.
7. Plank C *et al.* Activation of the complement system by synthetic DNA complexes: a potential barrier for intravenous gene delivery. *Hum Gene Ther* 1996; **7:** 1437-46.
8. Wagner E *et al.* Transferrin-polycation conjugates as carriers for DNA uptake into cells. *Proc Natl Acad Sci U S A* 1990; **87:** 3410-4.
9. Ogris M *et al.* PEGylated DNA/transferrin-PEI complexes: reduced interaction with blood components, extended circulation in blood and potential for systemic gene delivery. *Gene Ther* 1999; **6:** 595-605.
10. Smith RM, Wu GY. Hepatocyte-directed gene delivery by receptor-mediated endocytosis. *Semin Liver Dis* 1999; **19:** 83-92.
11. Ferkol T, Kaetzel CS Davis PB. Gene transfer into respiratory epithelial cells by targeting the polymeric immunoglobulin receptor. *J Clin Invest* 1993; **92:** 2394-400.
12. Hart S. Use of adhesion molecule for gene delivery. *Exp Nephrol* 1999; **7:** 193-9.
13. Shewring L *et al.* A nonviral vector system for efficient gene transfer to corneal endothelial cells via membrane integrins. *Transplantation* 1997; **64:** 763-9.
14. Sivolapenko GB *et al.* Imaging of metastatic melanoma utilising a technetium-99m labelled RGD- containing synthetic peptide. *Eur J Nucl Med* 1998; **25:** 1383-9.
15. Herard AL *et al.* Fibronectin and its alpha 5 beta 1-integrin receptor are involved in the wound-repair process of airway epithelium. Am *J Physiol* 1996; **271:** L726-33.
16. Pilewski JM *et al.* Expression of integrin cell adhesion receptors during human airway epithelial repair in vivo. *Am J Physiol* 1997; **273:** L256-63.
17. Romanov V *et al.* Two novel probes reveal tubular and vascular Arg-Gly-Asp (RGD) binding sites in the ischemic rat kidney. *Kidney Int* 1997; **52:** 93-102.
18. Shih PT *et al.* Minimally modified low-density lipoprotein induces monocyte adhesion to endothelial connecting segment-1 by activating beta1 integrin. J *Clin Invest* 1999; **103:** 613-25.
19. Leong JM, Morrissey PE, Isberg RR. A 76-amino acid disulfide loop in the Yersinia pseudotuberculosis invasin protein is required for integrin receptor recognition. *J Biol Chem* 1993; **268:** 20524-32.
20. Dersch P, Isberg RR. A region of the Yersinia pseudotuberculosis invasin protein enhances integrin-mediated uptake into mammalian cells and promotes self-association. *Embo J* 1999; **18:** 1199-213.
21. Hart S *et al.* Lipid-mediated enhancement of transfection by a nonviral integrin-targeting vector. *Hum Gen Ther* 1998; **9:** 575-85.
22. Erbacher P, Remy JS, Behr JP. Gene transfer with synthetic virus-like particles via the integrin-mediated endocytosis pathway. *Gene Ther* 1999; **6:** 138-45.
23. Uherek C, Fominaya J, Wels W. A. modular DNA carrier protein based on the structure of diphtheria toxin mediates target cell-specific gene delivery. *J Biol Chem* 1998; **273:** 8835-41.
24. Paul RW *et al.* Gene transfer using a novel fusion protein, GAL4/invasin. *Hum Gene Ther 1997;* **8:** 1253-62.
25. Khadake JR, Manchanahalli R, Satyanaranaya R. Condensation of DNA and chromatin by an SPKK-containing octapeptide repeat motif present in the C-terminus of histone H1. *Biochemistry* 1997; **36:** 1041-1051.
26. Wilke M *et al.* Efficacy of a peptide-based gene delivery system depends on mitotic activity. *Gene Ther* 1996; **3:** 1133-42.
27. Gong Y *et al.* Structural analysis of the N- and C-termini in a peptide with consensus sequence. Protein *Sci* 1995; **4:** 1446-56.
28. Leong JM, Fournier RS, Isberg RR. Identification of the integrin binding domain of the Yersinia pseudotuberculosis invasin protein. *Embo J* 1990; **9:** 1979-89.
29. Van Nhieu GT, Isberg RR. The yersinia pseudotubercolosis invasin protein and human fibronectin bind to mutually exclusive sites on the a5b1 integrin receptor. *the journal of biological chemistry* 1991; **266:** 24367-24375.
30. Garcia AJ, Takagi J, Boettiger D. Two-stage activation for alpha5beta1 integrin binding to surface- adsorbed fibronectin. *J Biol Chem* 1998; **273:** 34710-5.
31. Hajjar RJ *et al.* Modulation of ventricular function through gene transfer in vivo. *Proc Natl Acad Sci U S A* 1998; **95:** 5251-6.
32. D'Souza ST, Ginsberg MH Plow EF. Arginyl-glycyl-aspartic acid (RGD): a cell adhesion motif. *Trends in biological sciences* 1991; **16:** 246-250.
33. Harbottle R *et al.* An RGD-olygolysine peptide: a prototype construct for integrin-mediated gene delivery. *Hum Gene Ther* 1998; **9:** 1037-47.
34. Eble J *et al.* Recombinant soluble a3bl integrin: purification, Processing,Regulation, and specific binding to laminin-5cand invasin in a mutually exclusive manner. *Biochemistry* 1998; **37:** 10945-10955.
35. Isberg RR, Leong JM. Multiple beta 1 chain integrins are receptors for invasin, a protein that promotes bacterial penetration into mammalian cells. *Cell* 1990; **60:** 861-71.
36. Leong J *et al.* An aspartate residue of yersinia pseudotubercolosis invasin protein that is critical for integrin binding. *Embo Journal* 1995; **14:** 422-431.
37. Cheng PW. Receptor ligand-facilitated gene transfer: enhancement of liposome- mediated gene transfer and expression by transferrin. *Hum Gene Ther 1996;* **7:** 275-82.
38. Brasaemle DL, Attie AD. Microelisa reader quantification of fixed, stained, solubilized cells in microtitre dishes. *BioTechniques* 1988; **6:** 418-419.
39. de Wet JR *et al.* Firefly luciferase gene: structure and expression in mammalian cells. *Mol Cell Biol* 1987; **7:** 725-37.
40. Hamburger ZA *et al.* Crystal structure of invasin: a bacterial integrin binding protein. *Science* 1999; **286:** 291-5.
41. Milner, R., M. Wilby, S. Nishimura, K. Boylen, G. Edwards, J. Fawcett, C. Streuli, R. Pytela, and C. ffrench-Constant. 1997. Division of labor of Schwann cell integrins during migration on peripheral nerve extracellular matrix ligands. Dev Biol. **185:**215-28.
42. Feltri, M. L., S. S. Scherer, R. Nemni, J. Kamholz, H. Vogelbacker, M. O. Scott, N. Canal, V. Quaranta, and L. Wrabetz. 1994. Beta 4 integrin expression in myelinating Schwann cells is polarized, developmentally regulated and axonally dependent. Development. **120:**1287-301.

## Claims

1. A gene delivery vehicle comprising a general nucleic acid binding domain and an integrin binding domain.

2. A gene delivery vehicle according to claim 1, which comprises a fusion-protein of said nucleic acid binding domain and said integrin binding domain.

3. A gene delivery vehicle according to claim 1 or 2, wherein said integrin binding domain binds β1-integrin.

4. A gene delivery vehicle according to claim 3, wherein said integrin binding domain is derived from the C-terminal region of the *Yersinia pseudotuberculosis* invasin or a functional homologue thereof.

5. A gene delivery vehicle according to any one of claims 1-4, wherein said nucleic acid binding domain is a DNA-binding domain.

6. A gene delivery vehicle according to claim 5, wherein said DNA-binding domain is derived from a histon DNA-binding domain, in particular from (SPKR)4 or a functional homologue thereof.

7. A gene delivery vehicle according to any one of claims 1-6, wherein said domains are connected through a rigid alpha-helical linker.

8. A gene delivery vehicle according to claim 7, wherein said domains are coupled to said linker through flexible hinge sequences.

9. A complex of a gene delivery vehicle according to any one of claims 1-8, associated with a nucleic acid comprising a gene of interest to be delivered.

10. A complex according to claim 9, wherein said nucleic acid comprising said gene of interest is a plasmid.

11. A composition for delivery of a gene of interest to a cell, comprising a complex according to claim 9 or 10 and a suitable excipient.

12. A composition according to claim 11, further comprising a lysogenic lipid.

13. A composition according to claim 12, wherein said lysogenic lipid comprises DOTAP.

14. A composition according to any one of claims 11-13, further comprising a polykation.

15. A composition according to claim 14, wherein said polykation comprises polyethylene-imine.

16. A composition according to claim 14 or claim 15, wherein said polykation is covalently linked to said general nucleic acid binding domain and/or said integrin binding domain.

17. A nucleic acid encoding a gene delivery vehicle according to any one of claims 1-8.

18. A nucleic acid according to claim 17, which comprises a part of a viral genome.

19. A gene delivery vehicle according to any one of claims 1-8, which is based on a viral particle.

20. A gene delivery vehicle according to claim 19, which comprises an adenoviral particle, a retroviral particle or an adeno-associated virus particle.

21. A production system for a gene delivery vehicle, complex, and/or composition according to any one of claims 1-16, 19, 20.
